(19) Europäisches Patentamt
European Patent Office
Office européen des brevets

(11) Publication number: 0 032 620
B2

(12) # NEW EUROPEAN PATENT SPECIFICATION

(45) Date of publication of the new patent specification: 04.02.87

(21) Application number: 80304528.5

(22) Date of filling: 16.12.80

(51) Int. Cl.⁴: C 07 C 69/616, C 07 C 69/612, C 07 C 69/38, C 07 C 67/32, C 07 C 79/46, C 07 C 85/11, C 07 C 101/44

(54) Process for preparing therapeutic 2-arylpropionic acids and esters, and novel arylmethylmalonate esters.

(30) Priority: 19.12.79 US 105063
19.12.79 US 105166

(43) Date of publication of application: 29.07.81 Bulletin 81/30

(45) Publication of the grant of the patent: 30.05.84 Bulletin 84/22

(45) Mention of the opposition decision: 04.02.87 Bulletin 87/6

(84) Designated Contracting States: BE CH DE FR GB IT LI NL SE

(56) References cited:
CH-A-574 921
CH-A-574 924
FR-A-2 289 180
GB-A-1 469 700
GB-A-1 514 082
US-A-2 080 057
US-A-3 755 427
US-A-3 868 391

Chemical Abstracts, vol. 86, 1977 Columbus, Ohio, USA M. MAKOSZA et al. "Reactions of organic anions. LVIII. The problem of competitive nucleophilic substitution of ortho- and para-halogens in 2,4-dihalonitrobenzenes by some carbanions" page 442, column 2, Abstract no. 155340k
Carney et al., Experientia 29, 938 (1973)
M. Perchinunno et al., Org. Prep. Proc. Int. 9, 303 (1977)
M. Makosza et al., Roczniki Chemii, Ann. Soc. Chim. Polonorus 50, 1841 (1976)

(73) Proprietor: THE UPJOHN COMPANY, 301 Henrietta Street, Kalamazoo, Michigan 49001 (US)

(72) Inventor: Hylton, Thomas Anthony, 126 South Fletcher Street, Kalamazoo Michigan (US)
Inventor: Walker, Jerry Arnold, 6170 Thunder Bluff Road, Kalamazoo Michigan (US)

(74) Representative: Perry, Robert Edward, GILL JENNINGS & EVERY 53- 64 Chancery Lane, London WC2A 1HN (GB)

(56) References cited: (continuation)
P. Sykes, Reaktionsmechanismen der Organischen Chemie, Verlag Chemie, Weinheim, 6. Auflage 1974, S. 257
H.R. Christen, Grundlagen der Organischen Chemie, Verlage Sauerländer, Diesterweg-Salle, Aarau, Frankfurt am Main, 2. Auflage 1972, S. 251
H. Beyer/W. Walter, Lehrbuch der Organischen Chemie, S. Hirzel Verlag, Stuttgart, 18. Auflage 1978, S. 283
Houben-Weyl, Methoden der Organischen Chemie, Band XIII/1, Metallorganische Verbindungen, Georg Thieme Verlag, Stuttgart, 4. Auflage 1970, S. 291, 292, 301, 325, 346, 350, 375
D. White, Synthetic Comm. 7 (8), 559 (1977)
A.P. Krapcho et al., Tetrahedron Letters 957 (1973)
R.B. Davis et al., J. Org. Chem. 25, 1884 (1960)
A. Takeda et al., J. Org. Chem., 38, 4148 (1973)
U. Schöllkopf et al., Liebigs Ann. Chem. 766, 116 (1972)
S. Bank, J. Org. Chem 37, 114 (1972)
R.M. Magid et al., Tetrahedron Lett. 47, 4877 (1968)
J. Bourdais et al., C.R. Acad. Sc. Paris, 263 (Serie C) 84 (1966)
Organikum, Autorenkollektiv, VEB Deutscher Verlag der Wissenschaften, Berlin, 13. Auflage, 1974, S. 371.

EP 0 032 620 B2

LIBER, STOCKHOLM 1987

## Description

This invention relates to processes for preparing theapeutic 2-arylproplonic acids and esters, and to certain novel arylmethylmalonate esters.

Flurbiprofen, i.e. 2-(2-fluoro-biphenylyl)propionic acid, has anti-inflammatory activity which is about 240 times that of aspirin and analgesic activity which is about 180 times that of aspirin, in standard laboratory tests. Despite this high activity, the toxicity ($LD_{50}$) of flurbiprofen is only 1.2-2.4 times greater than that of aspirin, in standard laboratory tests.

Diethyl 2-(3-chloro-4-nitrophenyl)-2-methylmalonate and diethyl 2-(2-chloro-4-aminophenyl)-2-methylmalonate are known as intermediates in the preparation of tertiary amino acid derivatives of 2-(3-chloro-4-aminophenyl)-2-propionic acid having anti-inflammatory properties. See Carney et al., "A Potent Non-Steroidal Anti-Inflammatory Agent: 2-[3-chloro-4-(3-pyrrolinyl)phenyl]propionic acid" Experientia 29/8, p 938 (August 15, 1973); and British Patent Specification No. 1,31 6,31 2 partially corresponding to Swiss Patent Specifications Nos. 574,521--2, 574,524--6. In this context, particular note should be taken of Example 1 of Swiss Patent Specification No. 574,924 and also of Makosza et al., 'Reactions of Organic Anions. Part LVIII. The problem of Competitive Nucleophilic Substitution of Ortho- and Para-Halogens in 2,4-Dihalo Nitrobenzenes by some Carbanions-', Roczniki Chemii Ann. Soc. Chim. Poionorum 50, p. 1 850 (1976).

U.S. Patent Specifications Nos. 2,755,427 and 3,793,457 disclose a 9-step synthesis of flurbiprofen, starting from 4-bromoacetophenone. Other syntheses are disclosed in, for example, U.S. Patent Specifications Nos. 3,784,705; 3,901,806; 3.959,364 and 3,624,142; Belgian Patent Specification No. 840,354; German Patent Specifications Nos. 2,533,397 and 2,613,812; and Japan Kokai 75.40,540 (1975). In these published methods, a number of intermediates are involved and/or potentially hazardous reactions or intermediates are employed, e.g. the Schiemann reaction for the introduction of fluorine.

Makosza et al, supra, discuss the use of aqueous sodium and potassium hydroxide in chloride displacement by 2-phenylaikanenitriles and phenylacetonitriles, but it is noted that there is no suggestion to extend the use of these bases to the similar chloride displacement, by malonate, which is also discussed. In fact, aqueous sodium hydroxide and potassium cabonate would be expected to interfere with the malonate-chloride displacement reaction by hydrolysing the malonate. Surprisingly, it has been found that good results can be obtained for displacements by malonate e.g. by using essentially non-aqeous alkali metal hydroxide or potassium carbonate. This has the advantage of employing easily handled bases.

The methods of preparing biaryls described herein are coupling processes which improve the known Comberg or Gomberg-Bachmann reaction discussed by March in Advanced Organic Chemistry: Reactions, Mechanisms, and Structure, pages 550-551, 1969 (McGraw-Hill, Inc.). However, March noted that "yields are not high (usually under 40%) because of the many side reactions undergone by diazonium salts" which are described as intermediates therein. Cadogan, in J. Chem. Soc., page 4257 11 562), discloses the use of pentyl nitrite as the diazotising agent with increasing yields of the named biaryls. Other more recent references. such as Dutch Published Patent Application No. 6,500.865, 7/26/75; C.A. 64,5005e (1566), and U.S. Patent Specification No. 3,592,455, disclose various coupling reactions based on the Gomberg or Gomberg-Bachmann citations.

According to a first aspect of the present invention, a process for preparing a compound of formula I

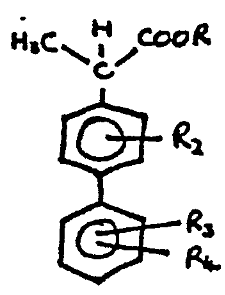

I

wherein R is hydrogen or $C_{1-6}$ alkyl; $R_2$ is hydrogen, halogen, $C_{1-6}$ alkoxy, $C_{1-6}$alkoxy)carbonyl, $C_{1-6}$alkyl, $C_{4-7}$ cycloalkyl, phenyl, cyano or nitro; and $R_3$ and $R_4$ are independently selected from hydrogen, halogen, hydroxy, nitro, $C_{1-6}$ alkyl, $C_{1-6}$ alkoxy, ($C_{1-6}$ alkoxy)carbonyl, aryloxycarbonyl, phenyl, cyano and $C_{4-7}$ cycloalkyl; comprises the steps of (1) reacting a compund of formula II

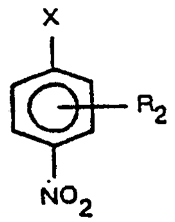

II

wherein $R_2$ is as defined above and X is halogen, with a methylmalonate ester of the formula

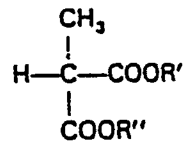

wherein either R' and R" are the same or different $C_{1-6}$ alkyl groups or R' and R" together are alkylene, in the presence of a base and under substantially non-aqueous conditions, to obtain a compund of formula III

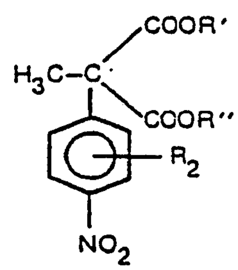

III

wherein $R_2$, R' and R" are as defined above;
(2) reducing the compund of formula III, to obtain a compound of formula IVX

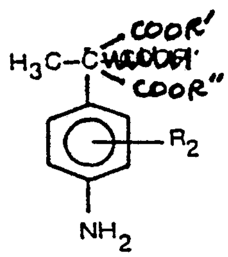

IVX

wherein $R_2$, R' and R" are as defined above;
(3) coupling the compound of formula IV with a compound of formula V

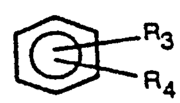

wherein $R_3$ and $R_4$ are as defined above, to obtain a compound of formula VI

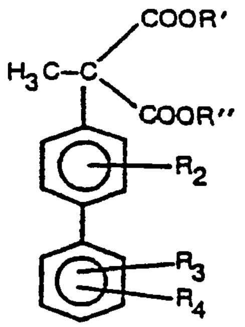

VI

wherein R' R'', $R_2$, $R_3$ and $R_4$ are as defined above; and

(4) decaboxylating the compound of formula VI and, if desired, hydrolysing ester to the acid.

According to of the invention. an alternative process for preparing a compound of formula I as defined above comprises conducting step (1) as defined above;

(1a) decaboxylating the compound of formula III, to obtain a compound of formula III'

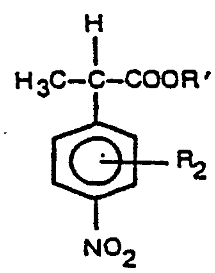

III'

wherein $R_2$ is as defined above and R' is $C_{1-6}$ alkyl; II (2) reducing the compound of formula III', to obtain a compound of formula IV'

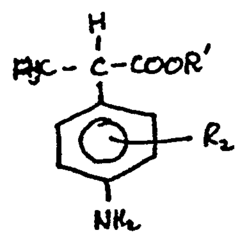

IV'

wherein $R_2$ and R' are as defined above; and

(3) coupling the compound of formula IV' with a compound of formula V as defined above and, if desired. hydrolysing the acid ester to the acid.

Novel compounds according to the invention are of formulae IIIa and IVa

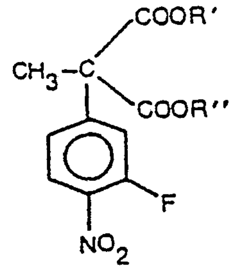

IIIa

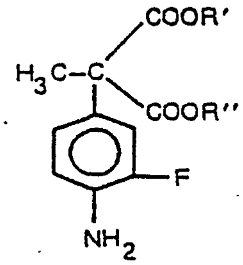

IVa

wherein R' and R" are as defined above, and the mono- and di-acid forms thereof. Examples of these novel compounds are diethyl 2-(3-fluorophenyl-4-nitro)-2-methylmalonate and diethyl 2-(4-amino-3-fluorophenyl)-2-methylmalonate.

The terms "$C_{1-6}$ alkyl" and "$C_{1-6}$ alkoxy" used herein refer to groups containing alkyl radicals which are, for example, methyl, ethyl, n-propyl, isopropyl, n-butyl, isobutyl, sec-butyl, tert-butyl, npentyl, isopentyl, neopentyl, n-hexyl or an isomer thereof. Any cyclic diester which R' and R" form may be, for example, similar to 2,2-dialkyl-1,3-dioxane-4,6-dione. "$C_{4-7}$ cycloalkyl" includes, for example, cyclobutyl, cyclopentyl, cyclohexyl and cycloheptyl. "Halogen" means fluorine, chlorine, bromine or iodine.

In step (1) of the novel process, the reaction of the 4-halonitrobenzene of formula II with the methylmalonate ester involves the selective displacement of X. When $R_2$ is the same as X, displacement at X occurs preferentially because X is at the para-position with respect to the nitro substituent. In general, it is preferred that teh compound of formula II has the formula

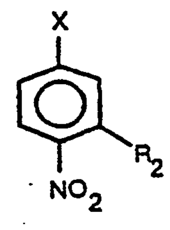

wherein $R_2$ and X are as defined above and, if $R_2$ is a halogen atom different from X, the ease of displacement of one halogen atom in the presence of another makes it preferable that the $R_2$ halogen atom should be no more reactive than X, and most preferably less reactive than X, with respect to the methylmalonate ester. The ease of displacement for X with respect to $R_2$ as halogen, ranked in order of least to most difficult to displace, is as follows:

$F >> Cl > Br > I$

The preferred compound of formula 11 is 2,4-difluoronitrobenzene.

The reaction of step (1) of the novel process is carried out in the presence of a base which may be. for example, sodium hydride (which has been used in the art). Preferably, the base is an alkaline metal hydroxide such as sodium or potassium hydroxide, or potassium carbonate. Sodium hydroxide is most preferred, e.g. in solid form such as pellets, flakes or powder.

The reaction is preferably conducted in an aprotic polar solvent such as dimethylformamide, dimethyl sulfoxide, sulfolane or hexamethylphosphoric triamide. The reaction temperature is preferably from 0 to 100, more preferably 25 to 35°C. A reaction temperature of from 25 to 35°C, and a reaction time of 2 to 5 hours, are preferred when using sodium hydroxide as the base. More rigorous reaction conditions, i.e. longer reaction times or elevated temperatures, are required when using potassium carbonate as the base. The reactants give high yields of compounds 111 when used in equimolar quantities and as 0.75--0.88 molar solutions: however, other concentrations can be used. Generally, a very slight excess of both base and, say, diethyl methylmalonate is preferred.

The use of strictly anhydrous conditions or a preformed enolate is unnecessary in this displacement reaction. However, the use of solvents such as methylene chloride and toluene in phase-transfer conditions, previously disclosed for similar displacements by 2-phenylalkanenitriles and phenylacetonitriles, is not effective for reactions carried out in the presence of sodium or potassium hydroxide and potassium carbonate bases.

Isolation of the compound 111, if desired, is accomplished by dilution of the reaction mixture with water, and extraction with an organic solvent, e.g. ethyl acetate. Purification is unnecessary before the reduction step, when it is desired to obtain the preferred fluoro-substituted compound IVa.

When it is desired to decarboxylate a formula 111 compound, this can be achieved by procedures similar to those described in the art, or as generally described herein. For example, decarboxylation may be achieved by reacting the formula 111 compound with an alkali metal fluoride to obtain a formula 111' compound, by procedures similar to those described in U.S. Patent Specification No. 3,240,824 and British Patent Specifications Nos. 1,469,700 and 1,514,082.

The reduction of the nitro group in compound 111 or 111' can be accomplished by known methods such as catalytically with hydrogen and a noble metal catalyst. or chemically using any one of a variety of well-known reducing agents such as iron or tin in an aqueous acidic medium, with sodium dithionite or with zinc in acetic acid.

Catalytic reduction with hydrogen may be carried out by standard methods, e.g. in a Parr apparatus with compound 111 in solution. A variety of solvents can be used, such as lower alkanols, aliphatic hydrocarbons having a boiling point over 35°C, chlorocarbons, ethyl acetate or aromatic liquid hydrocabons. A variety of standard noble metal hydrogenation catalysts can be used; palladium or platinum on carbon is preferred. Hydrogen pressures of from 1 to several bars can be used. Filtration removes the catalyst and the resulting compound IV in solution may be used without further purification in the coupling step described herein as step (3).

Chemical reduction can also be carried out, for example, with powdered iron in a water-ethanol mixture in

the presence of ammonium chloride. High conversion can be achieved at temperatures of about 50-80°C. Best results for obtaining compound IV are obtained by catalytic hydrogenation. Since the compound IV amines are sensitive to air in both the above reductions, an inert, e.g. nitrogen. atmosphere should be used.

In step (1) sodium hydride in a polar solvent underthe reaction conditions of the prior art may be used to accomplish the displacement reaction. However, these reactants and the conditions required by their use cause the displacement reaction to be less economical, more hazardous and more inconvenient than the cond.itions now found to be useful ent invention.

The coupling reaction of step (3) is conducted in order obtain a compound of formula I, the preferred examples of which are 2-(2-fluoro-4-biphenylpropionic acid, also known as flurbiprofen, and its ethyl ester. Within the generality of formula I, the aryl group in "aryloxycabonyl" is, for example, phenyl or phenyl substituted with, for example, one or more halogen atoms.

The coupling reaction may be conducted by the known Gomberg-Bachmann reaction, described above. Improvements may be achieved, however.

In a first preferred embodiment of the coupling reaction, a benzene solution of compound IV (or IV') is added simultaneously with an acid to a non-aqueous mixture of excess compound V and solid sodium or potassium nitrite. Alternatively, a benzene solution of compound IV is added simultaneously with an acid to a mixture of excess compound V and an aqueous solution of sodium or potassium nitrite. The ratio of the amounts of compounds IV to V is preferably from 5:10 to 1:10. The acid may be a mineral acid such as sulfuric acid or an organic acid such as benzoic, chloroacetic, dichloroacetic, trichloroacetic, methanesulfonic or acetic acid. The temperature of the reaction mixture is maintained between 25°C and the boiling temperature of the mixture. Molar ratios of acid and sodium or potassium nitrite to compound IV are preferably each from 1:1 to 4:1 times, most preferably 2.5:1. In an especially preferred embodiment, the compound IV and acetic acid are added dropwise to a mixture of the aqueous or nonaqueous metal nitrite in the compound V. Stirring from 2 to 18 hours after the addition is completed at the preferred temperatures of the reaction is advantageous. The product VI is isolated by cooling the reaction mixture washing, evaporating, distilling, or other conventional procedures. A particularly simple and preferred workup is evaporation, dissolution in 85% aqueous sulfuric acid and extraction of the product into hexane. Crude product VI is obtained and further purification may not be necessary for use of the product in maxing compounds 1. On the other hand, nitro compounds may be by-products in this reaction, so it may be advantageous to reduce the reaction mixture by adding iron/hydrochloric acid, zinc/acetic acid mixtures or sodium dithionite, which converts these by-products to amines such that these can be removed from the product simply by washing with acid. Conditions for the reduction of nitro compounds are similar to those outlined by S.R. Faudler et al., in "Organic Functionai Group Preparation". Vol. 1, Academic Press. New York, 1968, p. 339.

If the coupling reaction is carried out in nonaqueous conditions with solid metal nitrite, it is also advantageous to add an absorbent for water, such as anhydrous magnesium sulfate, silica gel or Celite®. Furthermore, the use of potassium nitrite rather than sodium nitrite in this reaction gives a higher yield and is therefore among the preferred conditions for the anhydrous coupling reaction.

In addition to the above named acids for use in this reaction it is found that in the two phase aqueous reaction mixture hydrofluoric and fluoboric are effective. However, fluoboric acid ($HBF_4$) gives a particuiarly high and unexpected yield. If sulfuric acid is used a 10% solution is preferred.

In preparing the especially preferred diethyl 2-(2-fluoro-4-biphenylyl)-2-methyl malonate compound VI for use in subsequent reactions according to the novel methods described herein the temperature of the coupiing reaction is 25° to 80°C, and preferably 68°-70°C.

The alternative coupling reaction, also noted above, employs alkyl nitrites instead of sodium nitrite and is accomplished in the absence of water. In this reaction a solution of the compound IV in excess benzene is reacted with alkyl nitrite such as isoamyl nitrite in the presence of the compound V at 20 to 80°C over a period of 5 to 20 hours. Preferably in this embodiment of the invention, isoamyl nitrite and a soiution of the compound IV in benzene are each added dropwise separately but simultaneously over a period of about 20 hours to an excess amount of the compound V while maintaining the temperature in the range of 25°C to the boiling point of the solvent, preferably about 65°C. The product VI may be treated with a reducing agent end isolated in a manner analogous to that described above for the preferred coupling reaction using metal nitrite.

It is also understood that the above coupling reaction is not limited to the preferred process conditions described herein. For example, conditions similar to those described in U.S. Patent 3,992,459 may be used to react compounds V and IV above. These conditions include the use of copper or copper salt.

Of particular interest is Example 5 wherein diethyl 2-(4-amino-3-fluorophenyl)-2-methyl malonate is substituted for the corresponding reactant, 2,4-difluoroaniline. However, some disadvantages exist in the recovery of the copper and/or disposal thereof which may distinguish the coupling reaction preparing VI of this invention noted above fiom U.S. Patent 3,992,459. On the other hand, each of the noted coupling reactions disclosed herein may be further modified by conducting the reaction in the presence of copper. Especially preferred is the use of the nonaqueous medium with solid sodium or potassium nitrite described herein with copoer. This copper may be in the form of a copper powder or a copper salt. However, if a copper powder is chosen reaction conditions are used which assure a timely preparation of copper salt in situ.

The step 4 treatment of compound VI which converts it to the acid I can be carried out by any known decaboxylation and hydrolysis. For example, compound VI may be reacted with an alkali hydroxide to obtain a dialkali salt thereof, acidified to obtain a malonic acid, and heated to achieve decarboxylation resulting in the desired acid.

The first part of this process is preferably carried out in a nitrogen atmosphere with an alkali hydroxide in aqueous alkanol at 0--25°C. The alkali hydroxide can be lithium, sodium, or potassium with sodium preferred. Alkanols such as methanol. ethanpl, 1-propanol, 2-propanol can be used with aqueous ethanol preferred. The ratio of the alkali hydroxide to the diester VI is between 2.5 and 6 moles per mole of diester. After the saponification is complete, the reaction mixture is diluted and acidified with a mineral acid such as hydrochloric acid, dilute sulfuric acid, or an organic acid such as acetic acid. The thus produced free malonic acid is extracted with an organic solvent, e.g., ethyl acetate, methylene dichloride, and the like.

The extracted material is evaporated and the residue dissolved in glacial acetic acid and heated to reflux for 1 to 1 0 hours to effect decarboxylation. The resulting product is compound 1, which is isolated and purified by conventional methods, e.g. evaporation, extraction, crystallization and the like.

The hydrolysis of the ester and decarboxylation of the diacid can also be carried out simultaneously by refluxing the malonate VI with acetic acid and a strong mineral acid. A good mixture for this is about 2-3 volume parts of acetic acid with 1 part of 4N-8N aqueous hydrochloric acid. The reaction time for the refluxing process is about 18-48 hours, for which reason the earlier base hydrolysis is preferred.

On the other hand treatment of the compound VI which converts itto the ester or acid of formula 1 can also be carried out by methods known to convert malonates directly to the desired monoesters. with further hydrolysis if desired. A description of a similar conversion reaction may be found in J. Med. Chem. 10, 1 078 (1967): Tetrahedron Letters 21 5 (1967) or Tetrahedron Letters 957 (1973). The highest yields and most amenable conditions preferred for use here are found in the latter of the above references in which compound VI is heated to a temperature of from 50 to 200, preferably 140 to 180, °C in a salt-solvent system of sodium chloride in wet dimethyl sulfoxide. Carbon dioxide and alkanols are produced in the reaction. Approximately 2 moles of water per mole of compound VI are added to the dimethyl sulfoxide to make it wet, and a slight excess of sodium chloride is used.

Isolation of the desired compound of formula 1, acid or ester, is achieved by conventional methods chosen for advantages dependent on the properties of the product.

In the following, Examples 1 to 33 illustrate the reaction of step (1), Example 34 illustrates the decarboxylation of step (1 a), Examples 35 to 42 illustrate the reduction of step (2). Examples 43 and 44 illustrate the coupling reaction of step (3), and Example 45 to 48 illustrate the decarboxylation of Step (4).

Starting materials for preparing dialkyl 2-(3-fluoro-4nitrophenyl)-2methylmalonate and dialkyl 2-(3-fluoro-4-aminophenyl)-2-methylmalonate and for use in the processes of this invention are available or can be prepared by methods described in the prior art.

**Example 1**

Diethyl 2-(3-fluoro-4-nitrophenyl)-2-methylmalonate

A mixture of 50.0 g (0.31 mol) 2,4-difluoronitrobenzene , 56.1 g (0.32 mol) diethyl methylmalonate and 400 ml dimethylformamide is vigorously stirred. To the stirring mixture are added 13.1 g (0.33 moll sodium hydroxide, in one portion, at 25°C. Ice is used to keep the temperature of the reaction mixture at 25-30°C during the first hour. Thereafter, stirring is maintained for 3.5 hours. 800 ml of water are then added, the organic layers removed, and the aqueous layer extracted with three 1 80 ml portions of ethyl acetate-water.

The combined organic layer and extracts are washed with diluted sodium suifate (3 times; a total of 2 litres) and then with 100 ml of saturated sodium sulfate. The solution is then dried over anhydrous sodium suifate and concentrated in vacuo to give 1 14.0 g of diethyl 2-(3-fluoro-4-nitrophenyl)-2-methylmalonate as an oil which can be used as such in the next step.

In like runs, the diethyl 2-(3-fluoro-4-nitrophenyl)-2-methylmaionate is purified to give a light yellow oil of boiling range 149-151°C at about 26.3 Pa.

**Examples 2 to 33**

Product as Example 1

Other runs are carried out in a similar manner to Example 1. Table I, below, gives the reactants. The solvent was always dimethylformamide (10% aqueous DMF in Example 21). The reactant equivalents are based on one equivalent of 2,4-difluoronitrobenzene (DFNB). DEMM is diethyl 2-methyl-malonate. The $K_2CO_3$ was anhydrous.

Table I

| Example | DFNB (Molar Conc) | DEMM (eq.) | Base | Base (eq.) |
|---|---|---|---|---|
| 2 | 0.75 | 1.02 | Flaked NaOH | 1.03 |
| 3 | 0.75 | 1.02 | Flaked NaOH | 1.03 |
| 4 | 0.75 | 1.02 | Flaked NaOH | 1.03 |
| 5 | 0.75 | 1.02 | Powdered NaOH | 1.03 |
| 6 | 0.75 | 1.02 | Flaked NaOH | 1.03 |
| 7 | 0.75 | 1.02 | Flaked NaOH | 1.2 |
| 8 | 0.75 | 1.02 | Flaked NaOH | 1.2 |
| 9 | 0.75 | 1.2 | Flaked NaOH | 1.03 |
| 10 | 0.75 | 1.13 | Flaked NaOH | 1.2 |
| 11 | 0.88 | 1.02 | Flaked NaOH | 1.03 |
| 12 | 0.88 | 1.02 | Flaked NaOH | 1.2 |
| 13 | 0.88 | 1.02 | Flaked NaOH | 1.3 |
| 14 | 0.88 | 1.02 | Flaked NaOH | 1.2 |
| 15 | 1.26 | 1.02 | Flaked NaOH | 1.2 |
| 16 | 3.14 | 1.02 | Flaked NaOH | 1.2 |
| 17 | 0.38 | 2.0 | Flaked NaOH | 2.07 |
| 18 | 0.75 | 1.0 | $K_2CO_3$ (60 mesh) | 1.0 |
| 19 | 0.75 | 1.02 | $K_2CO_3$ (60 mesh) | 1.15 |
| 20 | 0.75 | 1.02 | $K_2CO_3$ (60 mesh) | 1.1 |
| 21 | 0.75 | 1.02 | $K_2CO_3$ (60 mesh) | 1.1 |
| 22 | 0.75 | 1.02 | $K_2CO_3$ (60 mesh) | 1.25 |
| 23 | 0.38 | 1.02 | $K_2CO_3$ (300 mesh) | 1.25 |
| 24 | 0.75 | 1.28 | $K_2CO_3$ (60 mesh) | 1.25 |
| 25 | 0.75 | 1.02 | $K_2CO_3$ (300 mesh) | 1.25 |
| 26 | 0.75 | 1.02 | $K_2CO_3$ (60 mesh) | 1.25 |
| 27 | 0.75 | 1.02 | $K_2CO_3$ (60 mesh) | 1.5 |
| 28 | 0.75 | 1.02 | $K_2CO_3$ (60 mesh) | 1.25 |
| 29 | 0.75 | 1.02 | $K_2CO_3$ (60 mesh) | 1.25 |
| 30 | 0.75 | 1.02 | $K_2CO_3$ (300 mesh) | 1.25 |
| 31 | 0.75 | 1.02 | $K_2CO_3$ (300 mesh) | 1.25 |
| 32 | 0.75 | 1.02 | $K_2CO_3$ (300 mesh) | 1.25 |
| 33 | 0.75 | 1.02 | $Na_2CO_3$ | 1.25 |

Table II below, gives the conditions and results. A temperature given as 25 means that was the initial temperature, the exotherm being uncontrolled; <30 means an initial temperature of 5-15°C with control maintaining the temperature below 30°C; 25-30 means an initial temperature of 5-15°C with control maintaining the temperature between 25 and 30° C throughout the reaction. The yields were determined by gas-liquid chromatography.

Further results were obtained by continuing the process of Examples 2, 4, 5. 14. 22. 24 and 25 to, respectively, 20, 20, 20, 20, 60, 53 and 43 hours. The respective initially read yields, as given in Table II, of 79, 84, 84, 74, 60, 51 and 73% had changed to 77, 79, 75, 81. 73 and 75%.

Table II

| Example | Temperature (°C) | Time (Hours) | Yield (%) |
|---|---|---|---|
| 2 | <30 | 5 | 79 |
| 3 | 24—30 | 6 | 88 |
| 4 | 25 | 6 | 84 |
| 5 | 25 | 6 | 84 |
| 6 | 50 | 6 | 72 |
| 7 | 25 | 3 | 87 |
| 8 | <30 | 3 | 80 |
| 9 | 25 | 6 | 81 |
| 10 | 25 | 2 | 73 |
| 11 | 25—30 | 6 | 87 |
| 12 | <30 | 16 | 89 |
| 13 | <30 | 16 | 74 |
| 14 | 25 | 1 | 74 |
| 15 | 25 | 1 | 63 |
| 16 | 25 | 1 | 54 |
| 17 | 25 | 20 | 60 |
| 18 | 25 | 20 | |
| 19 | 60 | 18 | 58 |
| 20 | 60 | 19 | 65 |
| 21 | 60 | 18 | 37 |
| 22 | 60 | 23 | 60 |
| 23 | 60 | 21 | 78 |
| 24 | 60 | 23 | 51 |
| 25 | 60 | 18 | 73 |
| 26 | 60 | 28 | 69 |
| 27 | 60 | 25 | 58 |
| 28 | 90 | 23 | 61 |
| 29 | 120 | 20 | 77 |
| 30 | 120 | 16 | 86 |
| 31 | 140 | 2.5 | 73 |
| 32 | 155 | 1 | 79 |
| 33 | 60 | 44 | 11 |

Examples 1 to 33 confirm that yields above 85% can be obtained.

**Example 34**

Ethyl-2-(3-fluoro-4-nitrophenyl)propionate

A mixture of 0.1 moles diethyl 2-(3-fluoro-4-nitrophenyl)-2-methylmalonate, 0.11 moles sodium chloride and 50 ml dimethyl sulfoxide is heated under nitrogen at about 130-180°C for about 6 hours. On cooling, the mixture is diluted with 100 ml water and 100 ml ethyl acetate. The layers are separated and the organic layer is washed twice with 25 ml water. After drying over sodium sulfate, the organic layer is concentrated in vacuo to give the title product.

**Example 35**

Diethyl 2-(4-amino-3-fiuorophenyl)-2-methylmalonate

To a solution of 41.6 g diethyl 2-(4-nitro-3-fluorophenyl)-2-methylmalonate in 21 0 ml ethyl acetate in a 500 ml Parr hydrogenation bottle are added 830 mg (2 wt%) of 5% palladium/carbon. The mixture is hydrogenated in a Parr apparatus at 25°C under' e hydrogen pressure of 68-82 MPa. for 2.5 hours. The catalyst is removed by filtration through diatomaceous earth and is washed with a few ml of ethyl acetate. The solvent is removed in vacuo to give diethyl 2-(4-amino-3-fluorophenyl)-2-methylmalonate as a viscous oil, 37.6 g (100% wt yield).

**Example 36**

Product as Example 35

To a solution of 9.86 g (31.51 mmol) diethyl 2-(4-nitro-3-fluorophenyl)-3-methylmalonate in 60 ml of 50% aqueous ethanol are added 10.0 g (186.92 mmol) solid ammonium chloride followed by 4.48 g (80.21 mmol, 2.55 equiv.) activated iron fillings (max. particle size 410 μ).

The resulting mixture is stirred under nitrogen in an oil bath at 60°C for 4.5 hours and allowed to stand at room temperature for 12 hours. The reaction mixture is filtered through diatomaceouos earth and the solids are washed several times with 50% aqueous ethanol. The combined filtrates are concentrated in vacuo to remove most of the ethanol and are then diluted with 100 ml water. Extraction with 2 x 50 ml ethyl acetate gives an organic fraction which is washed with dilute aqueous sodium sulfate and dried over sodium sulfate. Concentration in vacuo gives 8.44 g (94.6% yield) diethyl 2-(4-amino-3-fluorophenyl)-2-methylmalonate as a dark-brown oil which gives, by distillation, an almost colourless oil boiling at about 190°C at 130 Pa.

Since the amine in Examples 35 and 36 is air-sensitive (darkens on exposure), all manipulations can be carried out under nitrogen.

**Examples 37 to 42**

Product as Example 35

The procedure of Example 36 was repeated under various conditions, still using 50% aqueous ethanol as the solvent and a reaction temperature of 60°C, except that 25°C was used in Example 41. The reactants and results are given in Table III.

**Table III**

| Example | Reactant | Iron (Form) | Time (Hours) | Yield (%) |
|---|---|---|---|---|
| 37 | 12N HCl | activated | 6 | 80 |
| 38 | 12N HCl (20 mol%) | activated | 4 | 80 |
| 39 | 12N HCl (10 mol%) | powder | 4 | 80 |
| 40 | 6 eq. $NH_4Cl$ | activated | 5 | 95 |
| 41 | 6 eq. $NH_4Cl$ | activated | 20 | 95 |
| 42 | 6 eq. $NH_4Cl$ | powder | 5 | 95 |

The activated iron fillings used in Examples 36, 37, 38, 40 and 41 (max. size 410 μ) are obtained by treating 10 g iron filings with 2 ml 12N aqueous HCl, followed by drying under nitrogen; see Jenkins et al, IND. Chem. Eng. 22 (1930) 31.

**Example 43**

Diethyl 2-(2-fluoro-4-biphenylyl)-2-methylmalonate

In a one-liter round bottom flask is placed 18.34 g (0.266 mol., 2 equiv.) of sodium nitrite, 22 ml of water, and 210 ml of benzene. The mixture is placed in an oil bath at ~68-70°C and is stirred vigorously. A solution of 37.6 g of diethyl 2-(4-amino-3-fluorophenyl)-2-methylmalonate, 16.0 g 10.266 moi. 2 equiv.) of glacial acetic acid, and 70 ml of benzene are added from an addition funnel dropwise over ~6 hours. Heating is continued for 2 hours after addition is complete, and the mixture is then allowed to stand overnight. The resulting mixture is transferred to a one-liter separatory funnel and the benzene layer is washed with 3 x 100 ml of dilute aqueous sodium sulfate solution. Concentration in vacuo gives a dark brown viscous oil, 47.8 g.

The crude product is dissolved in 66.5 ml of aq. sulfuric acid (85% v/v) and the resulting solution is extracted with 4 x 500 ml hexane. The combined hexane extracts are washed with 4 x 500 ml 1 M aqueous sodium carbonate solution and 100 ml of saturated aqueous sodium sulfate. Removal of the solvent in vacuo gives 26.5 g of diethyl 2-(2-fluoro-4-biphenylyl)-2-methylmalonate as a light yellow viscous oil (58% yield overall from difluoronitrobenzene).

In a manner similar to Example 43, diethyl 2-(2-fluoro-4-biphenylyl)-2-methylmalonate under slightly varying conditions is obtained in yields varying from 53-61%.

**Example 44**

Product as Example 42

In a one-liter round-bottom flask are placed 250 ml of benzene. A solution of ~9.1 g (from 31.44 mmol) of diethyl 2-(3-fluoro-4-aminophenyl)-2-methylmalonate in 110 ml benzene is placed in one funnel and a solution of 10.1 g (86.5 mmol, 2.75 equiv.) isoamyl nitrite in 50 ml benzene is placed in the other. With vigorous stirring at ~58°C, 0.35 g (3,0 mmol) of isoamyl nitrite is added to the reaction flask. The solutions of amine and nitrite are added simultaneously over 7 hours. When addition is complete, stirring is continued for 4 hours at ~59°C and the mixture is then allowed to stand for 12 hours. The solvent is removed in vacuo and the resulting dark brown oil (12.0 g) is transferred to a 250 ml separatory funnel. Ice cold 85% (v/v) aqueous sulfuric acid (16 ml) is added and the mixture is extracted with 4 x 120 ml hexane. The combined hexane extracts are washed with 4 x 100 ml of 1 M aqueous sodium carbonate, 50 ml of saturated aqueous sodium sulfate solution, and are dried over anhydrous sodium sulfate. Concentration in vacuo gives 6.32 g of 2-(2-fluoro-4-biphenylyl)-2-methyl-malonate as a yellow oil (58.0% based on 2,4-difluoronitrobenzene).

In a manner similar to Example 44, diethyl 2-(2-fluoro-4-biphenylyl)-2-methylmalonate is obtained under varying conditions in a yield of 52-72%.

**Example 45**

Flurbiprofen

To a stirred solution of 7.58 g (22.0 mmol) of diethyl 2-(2-fluoro-4-biphenylyl)-2-methylmalonate in 50 ml of absolute ethanol, maintained under nitrogen gas at ~15°C (ice bath) are added 8.72 g (109.0 mmol. 5.0 mol -equiv.) of 50% aqueous sodium hydroxide. The temperature is maintained at <25°C for 6 hours. The resulting suspension is diluted with 150 ml water and the pH is adjusted to ~8.0 with dilute hydrochloric acid. The solution is transferred to a separatory funnel and is extracted with 3 x 60 ml of methylene chloride. The aqueous layer is acidified to pH <1 with concentrated hydrochloric acid and is then extracted with 200 ml of ethyl acetate in three portions. The combined extracts are dried over anhydrous sodium sulfate and concentrated in vacuo to give 5.9 g 2-(2-fluoro-4-biphenylyl)-2-methyl malonic acid. This material is dissolved in 15 ml of glacial acetic acid and the solution is heated to reflux for ~17 hours. Water (15 ml) is added and the solution is allowed to cool slowly with stirring and seeding. After 1 hour stirring at room temperature, the mixture is cooled with stirring to 0-5°C, then allowed to stand for ~15 hours. The product is isolated, washed with a few ml of cold 50% aqueous acetic acid, and then dried to give 4.67 g flurbiprofen, melting point 113°-115°C.

**Example 46**

Flurbiprofen

In a 200 ml round-bottom flask are placed 15.03 g (~43.7 mmol) of ethyl 2-(2-fluoro-4-biphenyl)-2-methylmalonate, 62 ml of glacial acetic acid, and 22 ml of 6N aqueous hydrochloric acid. The mixture is refluxed under nitrogen for 24 hours. On cooling to room temperature, the solution is diluted with 250 ml of water and the mixture is extracted with 4 x 50 ml of methylene chloride. The combined extracts are washed with 2 x 100 ml of water and then with 8 x 40 ml (0.2N) of aqueous potassium hydroxide soiution. Extracts 1-4 are combined. acidified to pH <1 with concentrated hydrochloric acid, and the resulting solution is extracted with 3 x50 ml of methylene chloride. The organic extracts are dried over sodium sulfate and concentrated in vacuo to give 7.13 g (38.8% overall) crude flurbiprofen. Extract 5 is backwashed with 3 x 15 ml methylene chloride, acidified with concentrated hydrochloric acid, and the resulting mixture is extracted with 3 x 20 ml methylene chloride to give 1.84 g (10.0%) crude flurbiprofen. Fractions 6-8 are combined and the pH is adjusted to ~19.0. After washing with 3 x 30 ml methylene chloride, the aqueous is acidified with concentrated hydrochloric acid and is then extracted with 3 x 50 ml of methylene chloride. Drying and concentration of the extracts gives 0.53 g (4.0%) crude flurbiprofen.

The combined crude flurbiprofen (9.5 g) is crystallized from 59 ml of 50% aqueous acetic acid as previously described to give 8.80 g of flurbiprofen (82,6% conversion from malonate; 47.9% overall from 2,4-difluoronitrobenzene).

**Example 47**

Flurbiprofen

In a 200 ml round-bottom flask are placed 10.0 g (~29.1 mmol) of diethyl 2-(2-fluoro-4-biphenylyl)-methylmalonate, 9.4 g (167.9 mmol, 6 equiv.) of solid potassium hydroxide, 33 ml of absolute ethanol, and 60 ml water. The resulting two-phase mixture is stirred under reflux for 17 hours. Most of the ethanol is removed in vacuo and the residue is diluted with 100 ml water. The pH is adjusted to ~8 with hydrochloric acid. The aqueous solution is extracted with 5 x 100 ml methylene chloride. The aqueous layer is acidified with 6N hydrochloric acid to a pH ~1 and is then extracted with 50 ml of methylene chloride and 3 x 50 ml of ethyl acetate. The combined organic extracts are washed with dilute aqueous sodium sulfate solution and dried over anhydrous sodium sulfate. Concentration in vacuo gives 6.38 g of tan solids. This material is dissolved in 12 ml of glacial acetic acid and the solution is heated to reflux for 17 hours at which time activated charcoal (Darco G-70) (600 mg) is added. After an additional 3 hours, the solution is filtered and the filtrate is diluted with water to give ca. 50% aqueous acetic acid solution. On seeding at ~80° C and further cooling. the product crystallizes and there are isolated 5.63 g (45.3% overall yield) of flurbiprofen.

**Example 48**

Ethyl 2-(2-fluoro-4-biphenylyl)propionate.

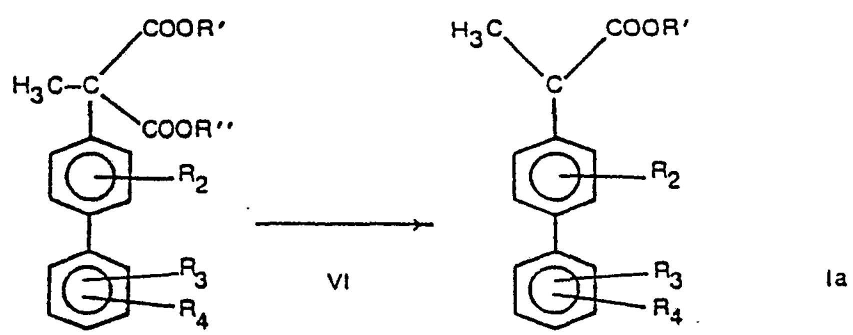

A mixture of 100 millimoles diethyl 2-(2-fluoro-4-biphenyl)-methylmalonate. 110 millimoles sodium chloride, 200 millimoles water and 50 milliliters dimethyl sulfoxide is heated under nitrogen at about 130° to 180°C for about 6 hours. On cooling, the mixture is diluted with 100 milliliters water and 100 milliliters ethyl acetate. The layers are separated and the organic layer is washed two times with 25 milliliters of water. After drying over disodium sulfate the organic layer is concentrated in vacuo to give the desired ethyl fluirbiprofen.

**Claims**

1. A process for preparing a compound of formula I

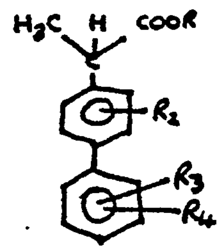

I

wherein R is hydrogen or $C_{1-6}$alkyl $R_2$ is hydrogen, halogen, $C_{1-6}$alkoxy, ($C_{1-6}$alkoxy)carbonyl, $C_{1-6}$alkyl, $C_{4-7}$ cycloalkyl, phenyl, cyano or nitro; and $R_3$ $R_4$ are independently selected from hydrogen, halogen, hydroxy, nitro, $C_{1-6}$ alkyl, $C_{1-6}$ alkoxy, ($C_{1-6}$ alkoxy)carbonyl, aryloxycarbonyl, phenyl, cyano and $C_{4-7}$ cycloalkyl; which comprises the steps of (1) reacting a compound of formula II

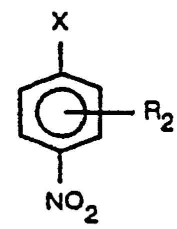

II

wherein $R_2$ is as defined above and X is halogen, with a methylmalonate ester of the formula

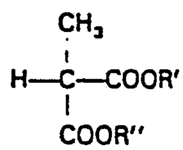

wherein either R' and R'' are the same or different $C_{1-6}$ alkyl groups or R' and R'' together are alkylene, in the presence of a base and under substantially non-aqueous Conditions, to obtain a compound of formula III

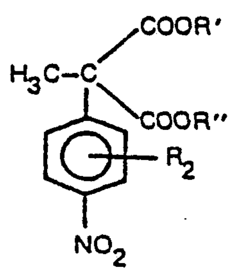

III

wherein $R_2$, R' and R'' are as defined above;
(2) reducing the compound of formula III, to obtain a compound of formula IV

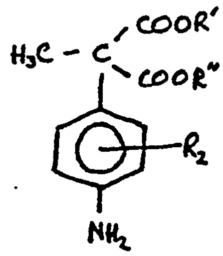

IV

wherein $R_2$, R' and R'' are as defined above;
(3) coupling the compound of formula IV with a compound of formula V

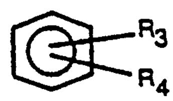

V

wherein $R_3$ and $R_4$ are as defined above, to obtain a compound of formula VI

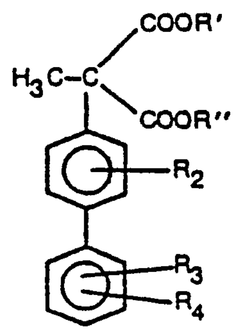

VI

wherein R', R'', $R_2$, $R_3$ and $R_4$ are as defined above; and
(4) decarboxylating the compound of formla VI and, if desired, hydrolysing the acid ester to the acid.

2. A process for preparing a compound of formula I as defined in claim 1; which comprises conducting step (1) as defined in claim 1:
(1a) decaboxylating the compound of formula III, to obtain a compound of formula III'

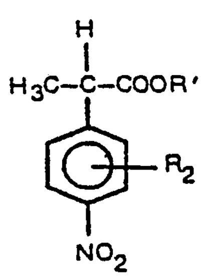

III'

wherein $R_2$ is as defined in claim 1 and R' is $C_{1-6}$ alkyl;
(2) reducing the compound of formula III', to obtain a compound of formula IV'

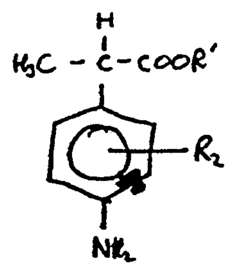

IV'

wherein $R_2$ and R' are as defined above; and

(3) coupling the compound of formula IV' with a compound of formula V as defined in claim 1 and, if desired, hydrolysing the acid ester to the acid.

3. A process according to claim 1 or claim 2, in which step (3) is conducted in the presence of copper.

4. A process according to any preceding claim, in which step (3) is conducted in the presence of aqueous metal nitrite and an acid.

5. A process according to claim 4, in which the compound of formula IV or IV' and the acid are added simultaneously to aqueous sodium nitrite in an excess of the compound of formula V, such that the weight ratio of sodium nitrite to the compound of formula IV or IV' is from 1:1 to 4:1 and the weight ratio of the acid to the compound of formula IV or IV' is 1:1 to 4:1.

6. A process according to claim 4 or claim 5, in which the acid is sulfuric, acetic, fluoboric or trichloroacetic acid

7. A process according to any of claims 1 to 3, in which step (3) is conducted in the presence of non-aqueous metal nitrite and acid.

8. A process according to claim 7, in which the compound of formula V and the acid are simultaneously added to a mixture of finely divided potassium nitrite in an excess of the compound of formula IV or IV' such that the weight ratio of potassium nitrite to the compound of formula IV or IV' is from 1:1 to 4:1 and the weight ratio of the acid to the compound of formula IV or IV' is from 1:1 to 4:1.

9. A process according to any of claims 1 to 3, in which step (3) is conducted by adding a alkyl nitrite and the compound of formula IV or IV' separately and simultaneously to an excess of the compound of formula V.

10. A process according to any preceding claim in which the compound of formula I is 2-(2-fluoro-4-biphenylyl)propionic acid or its ethyl ester.

11. A process according to any preceding claim, in which the base is an alkali metal hydroxide or potassium carbonate.

12. A process according to claim 11, in which the base is sodium hydroxide.

13. A process according to any preceding claim, in which step (1) is conducted in the presence of an aprotic solvent.

14. A process according to any preceding claim. in which step (1) is conducted at from 0 to 100° C. 14

15. A process according to claim 14, in which step (1) is conducted at from 25 to 35° C.

16. A process according to any preceding claim, in which the compound of formula II has the formula

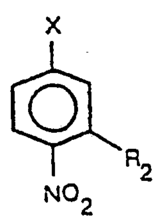

wherein $R_2$ and X are as defined in claim 1.

17. A process according to claim 16 wherein $R_2$ is a halogen atom no more reactiVe than X with respect to the methylmalonate ester.

18. A process according to claim 17 wherein $R_2$ is less reactive than X.

19. A process according to claim 16 in which the compound of formula II is 2,4-difluoronitro-benzene.

20. A compound of the formula

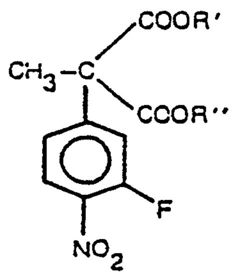

IIIa

wherein R' and R" are as defined in claim 1, or a mono- or di-acid form thereof.

21. Diethyl 2-(3-flouro-4-nitrophenyl)-2-methylmalonat

22. A compound of the formula

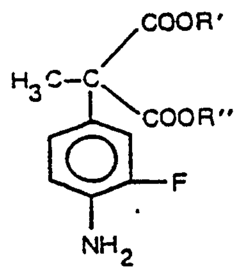

IVa

wherein R' and R" are as defined in claim 1, or a mono- or di-acid form thereof.

23. Diethyl 2-(4-amino-3-fluorophenyl)-2-methylmalonate.

## Revendications

1. Procédé de production d'un composé de formule I

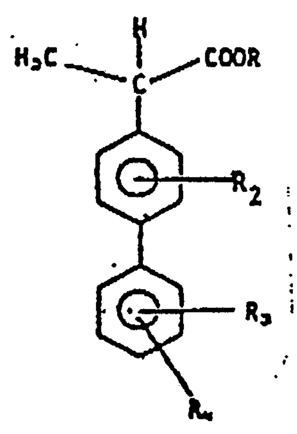

I

dans laquelle R est l'hydrogène ou un groupe alkyle en $C_1$ à $C_6$, $R_2$ est l'hydrogène, un halogène, un reste alkoxy en $C_1$ à $C_6$, (alkoxy en $C_1$ à $C_6$)carbonyle, alkyle en $C_1$ à $C_6$, cycloalkyle en $C_4$ à $C_7$, phényle, cyano ou nitro; et $R_3$ et $R_4$ sont choisis indépendamment entre l'hydrogène, un halogène, un groupe hydroxy, nitro, alkyle en $C_1$ à $C_6$, alkoxy en $C_1$ à $C_6$, (alkoxy en $C_1$ à $C_6$)carbonyle, aryloxycarbonyle, phényle, cyano et cycloalkyle en $C_4$ à $C_7$; procédé qui comprend les étapes consistant (1) à faire réagir un composé de formule II

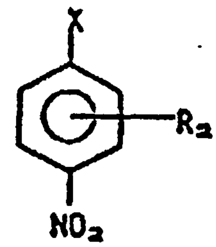

II

dans laquelle $R_2$ a la définition donnée ci-dessus et X est un halogène, avec un ester méthylmalonique de formula

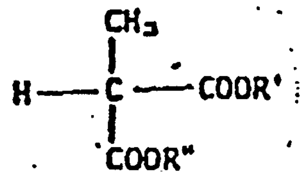

dans laquelle R' et R" sont des groupes alkyle en $C_1$ à $C_6$ identiques ou différents ou bien R' et R" forment ensemble un groupe alkylène, en présence d'une base et dans des conditione prétiquement non aqueuses, pour obtenir un composé de formule III

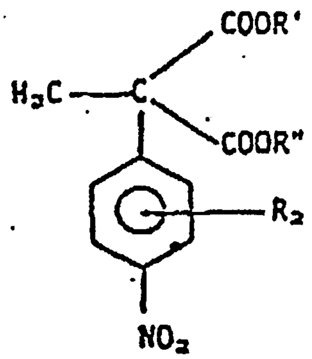

III

dans laquelle $R_2$, R' et R" ont las définitions données ci-dessus; (2) à réduire la composé de formule III, pour obtenir un composé de formule IV

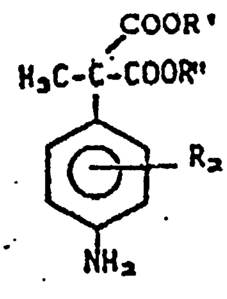

IV

dans laquelle $R_2$, R' et R'' ont la définition ci-dessus; (3) à combiner le composé de formule IV avec un composé de formule V

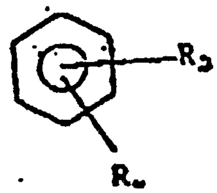

V

dans laquelle $R_3$ et $R_4$ ont la définition donnée ci-dessus pour obtenir un composé de formule VI

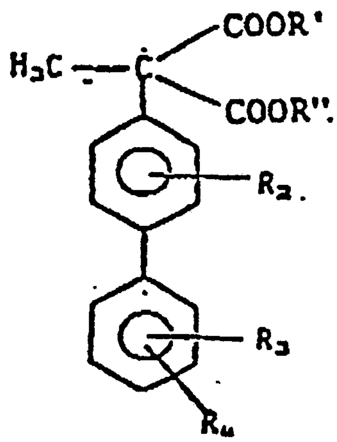

VI

dans laquelle R' R'', $R_2$, $R_3$ et $R_4$ ont les définitions données ci-dessus; et (4) à décarboxyler le composé de formule VI et, le cas échéant, à hydrolyser l'ester d'acide en l'acide.

2. Procédé de production d'un composé de formule I comme défini dans la revendication 1, qui consiste à conduire l'étape (1) comme défini dans la revendication 1; (1a) à décarboxyler le composé de formule III pour obtenir un composé de formule III,

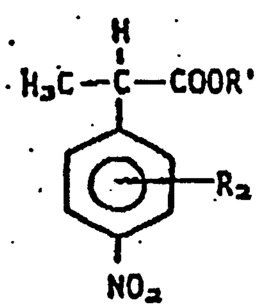

III'

dans laquelle $R_2$ est tel que défini dans la revendication 1, et R' est un groupe alkyle en $C_1$ à $C_6$; (2) à réduire le composé de formule III', pour obtenir un composé de formule IV,

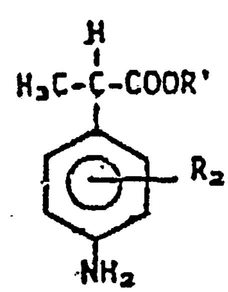

IV'

dans laquelle $R_2$ et R' sont tels que définis ci-dessus; et (3) à combiner le composé de formule IV' avec un composé de formule V comme défini dans la revendication 1 et, le cas échéant, à hydrolyser l'ester d'acide en l'acide.

3. Procédé suivant la revendication 1 ou la revendication 2, dans lequel l'étape (3) est conduite en présence de cuivre.

4. Procédé suivant l'une quelconque des revendications précédentes, dans lequel l'étape (3) est conduite en présence d'un nitrite métallique aqueux et d'un acide.

5. Procédé suivant la revendication 4, dans lequel le composé de formule IV ou IV' et l'acide sont ajoutés simultanément à du nitrite de sodium aqueux dans un excès du composé de formule V de manière que le rapport en poids du nitrite de sodium au composé de formule IV ou IV' ait une valeur de 1:4 à 4:1 et que le rapport en poids de l'acide au composé de formule IV ou IV' ait une valeur de 1:1 à 4:1.

6. Procédé suivant la revendication 4 ou la revendication 5, dans lequel l'acide est l'acide sulfurique, acétique, fluoroborique ou trichloracétique.

7. Procédé suivant l'une quelconque des revendications 1 à 3, dans lequel l'étape (3) est conduite en présence de nitrite métallique non aqueux et d'acide.

8. Procédé suivant la revendication 7, dans lequel le composé de formule V et l'acide sont ajoutés simultanément à un mélange de nitrite de potassium finement divisé dans un excès du composé de formule IV ou IV' de manière que le rapport en poids du nitrite de potassium au composé de formule IV ou IV' ait une valeur de 1:1 à 4:1 et que le rapport en poids de l'acide au composé de formule IV ou IV' ait une valeur de 1:1 à 4:1.

9. Procédé suivant l'une quelconque des revendications 1 à 3, dans lequel l'étape (3) est conduite par addition d'un nitrite d'alkyle en $C_3$ à $C_8$ et du composé de formule IV ou IV' séparément et simultanément à un excèes du composé de formule V:

10. Procédé suivant l'une quelconque des revendications précédentes, dans lequel le composé de formule I est l'acide 2,(2-fluoro-4-biphénylyl)propionique ou son ester éthylique.

11. Procédé suivant l'une quelconque des revendications précédentes, dans lequel la base est un hydroxyde de métal alcalin ou le carbonate de potassium. 12. Procédé suivant la revendication 11, dans lequel la base est l'hydroxyde de sodium.

13. Procédé suivant l'une quelconque des revendications précédentes, dans lequel l'étape (1) est conduite en présence d'un solvant aprotique.

14. Procédé suivant l'une quelconque des revendications précédentes, dans lequel l'étape (1) est conduite à une température de 0 à 100°C.

15. Procédé suivant la revendication 14, dans lequel l'étape (1) est conduite à une température de 25 à 35°C.

16. Procédé suivant l'une quelconque des revendications précédentes, dans lequel le composé de formule II répond à la formule

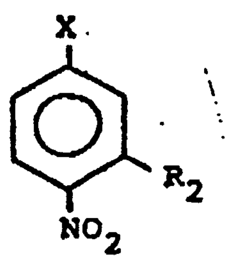

dans laquelle $R_2$ et X ont la définition donnée dans la revendication 1.

17. Procédé suivent la revendication 16, dans lequel $R_2$ est un atome d'halogène qui n'est pas plus réactif que X vis-à-vis de l'ester méthylmalonique.

18. Procédé suivant la revendication 17, dans lequel $R_2$ est moins réactif que X.

19. Procédé suivant la revendication 16, dans lequel le composé de formule II est le 2,4-difluoronitrobenzène.

20. Composé de formule

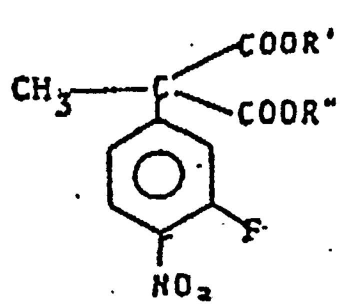

IIIa

dans laquelle R' et R'' ont la définition donnée dans la revendication 1, ou une forme monoacide ou diacide de ce composé.

21. Le 2-(3-fluoro-4-nitrophényl)-2-méthyl-malonate de diéthyle.

22. Composé de formule

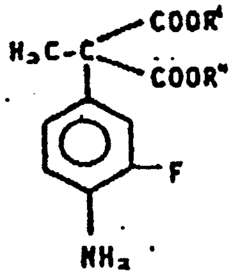

IVa

dans laquelle R' et R'' ont la définition donnée dans la revendication 1, ou une forme monoacide ou diacide de ce composé.

23. Le 2,(4-amino-3-fluorophényl)-2-méthyl-malonate de diéthyle.

**Patentansprüche**

1. Ein Verfahren zum Herstellen einer Verbindung der Formel I

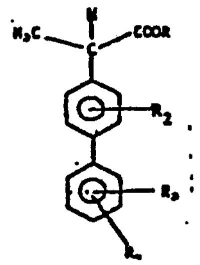

I

worin R Wasserstoff oder $C_{1-6}$ Alkyl ist; $R_2$ ist Wasserstoff, Halogen, $C_{1-6}$ Alkoxy, ($C_{1-6}$ Alkoxy) Karbonyl, $C_{1-6}$ Alkyl, $C_{4-7}$ Cycloalkyl, Phenyl, Cyano oder Nitro; und $R_3$ und $R_4$ sind unabhängig gewählt von Wasserstoff, Halogen, Hydroxy, Nitro, $C_{1-6}$ Alkyl, $C_{1-6}$ Alkoxy, ($C_{1-6}$ Alkoxy) Karbonyl, Aryloxykarbonyl, Phenyl, Cyano und $C_{4-7}$ Cycloalkyl; welches die Schritte (1) des Reagierens einer Verbindung der Formel II

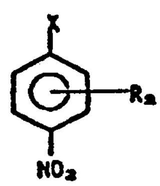

II

worin $R_2$ wie oben definiert ist und X ein Halogen ist, mit einem Methylmalonatester der Formel

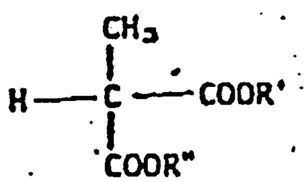

umfaßt, worin R' sowie R'' entweder gleiche oder verschiedene $C_{1-6}$ Alkylgruppen sind oder R' und R'' zusammen Alkylen ist, in Gegenwart einer Base und unter wesentlich nicht wässerigen Bedingungen, um eine Verbindungen der Formel III

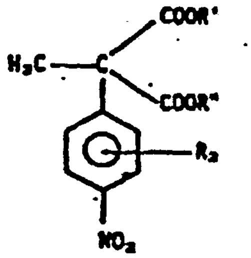

III

zu erstellen, worin $R_2$, R' und R'' wie oben definiert sind;

(2) des Reduzierens der Verbindung der Formel III umfaßt, um eine Verbindung der Formel IV

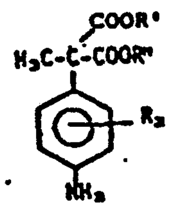

IV'

zu erstellen, worin $R_2$, R' und R'' wie oben definiert sind;

(3) Koppeln der Verbindung der Formel IV mit einer Verbindung der Formel V

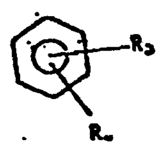

V

worin $R_3$ und $R_4$ wie oben definiert sind, um eine Verbindung der Formel VI

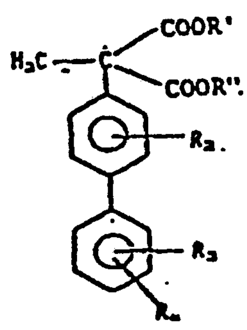

VI

zu erstellen, R', R'', $R_2$, $R_3$ und $R_4$ wie oben definiert sind; und

(4) Dekarboxylieren der Verbindung der Formel VI und, falls gewünscht, Hydrolisieren des Säureesters zur Säure.

2. Ein Verfahren zum Herstellen einer Verbindung der Formel I wie in Anspruch 1 definiert ist; welches das Ausführen des in Anspruch 1 definierten Schrittes (1) umfaßt;

(1a) Dekarboxylieren der Verbindung der Formel III, um eine Verbindung der Formel III' zu erhalten

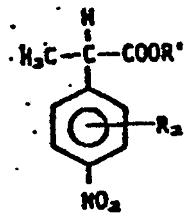

III'

worin $R_2$ wie in Anspruch 1 definiert ist und R' $C_{1-6}$ Alkyl ist;
(2) Reduzieren der Verbindung der Formel III', um eine Verbindung der Formel IV' zu erhalten,

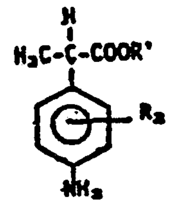

IV'

worin $R_2$ und R' wie oben definiert sind; und
(3) Koppeln der Verbindung der Formel IV' mit einer Verbindung der Formel V wie in Anspruch 1 definiert ist, und, falls gewünscht, Hydrolisieren des Säureesters zur Säure.

3. Ein Verfahren nach Anspruch 1 oder Anspruch 2, worin der Verfahrensschritt (3) in Anwesenheit von Kuperausgeführt wird.

4. Ein Verfahren nach einem der vorhergehenden Ansprüche, worin der Verfahrensschritt (3) in Anwesenheit von wässerigem Metallnitrit und einer Säure ausgeführt wird.

5. Ein Verfahren nach Anspruch 4, worin die Verbindung der Formel IV oder IV' und die Säure gleichzeitig zu wässerigem Natriumnitrit in einem Überschuß der Verbindung der Formel V zugegeben werden, sodaß das Gewicht sverhältnis zwischen Natriumnitrit und der Verbindung der Formel IV oder IV' sich zwischen 1: 1 und 4: 1 erstreckt und das Gewichtsverhältnis zwischen der Säure und der Verbindung der Formel IV oder IV' sich zwischen 1:1 und 1:4 erstreckt.

6. Ein Verfahren nach Anspruch 4 oder 5, worin die Säure Schwefel-, Essig-, Fluobor- oder Trichloressigsäure ist.

7. Ein Verfahren nach einem der Ansprüche 1 bis 3, worin der Verfahrensschritt (3) in Anwesenheit von von nichtwässerigem Metallnitrit und Säure ausgeführt wird.

8. Ein Verfahren nach Anspruch 7, worin die Verbindung det Formel V und die Säure gleichzeitig einem Gemisch von Kaliumnitrit in einem Überschuß der Formel IV oder IV' derart zugegeben werden, daß das Gewichtsverhältnis zwischen Kaliumnitrit und der Verbindung der Formel IV oder IV' von 1: 1 bis 4: 1 und das Gewichtsverhältnis zwischen der Säure und der Verbindung der Formel IV oder IV' 1:1 bis 4: 1 beträgt.

9. Ein Verfahren nach einem der ansprüche 1 bis 3, worin der Verfahrensschritt (3) durch Zufügen eines $C_{3-8}$ Alkylnitrits und der Verbindung der Formel IV oder IV', gesondert und gleichzeitig, zu einem Überschuß der Verbindung der Formel V ausgeführt wird.

10. Ein Verfahren nach einem der vorhergehenden Anspruche, worin die Verbindung der Formel I 2-(2-Fluoro-4-Biphenylyl) Proprionsäure oder ihr Ethylester ist.

11. Ein Verfahren nach einem der vorhergehenden Ansprüche, worin die Base ein alkalisches Metallhydroxyd oder Kaliumkarbonat ist.

12. Ein Verfahren nach Anspruch 11, in welchem die Base Natrium hydroxid ist.

13. Ein Verfahren nach einem der vorhergehenden Ansprüche, in dem der Verfahrensschritt (1) in der Anwesenheit eines aprotischen Lösungsmittels ausgeführt wird.

14. Ein Verfahren nach einem vorhergehenden Anspruch, in dem Schritt (1) bei von 0 bis 100° ausgeführt wird.

15. Ein Verfahren nach Anspruch 14, in dem Schritt (1) bei von 25 bis 35° C ausgeführt wird.

16. Ein Verfahren nach einem der vorhergehenden ansprüche, in dem die Verbindung der Formel II die Formel

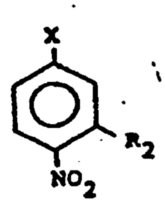

aufweist, worin $R_2$ und X wie in Anspruch 1 definiert sind.

17. Ein Verfahren nach Anspruch 16, worin $R_2$ ein Halogenatom ist, welches in Bezug auf den Methylmalonatester nicht mehr als X reaktiv ist.

18. Ein Verfahren nach Anspruch 17, worin $R_2$ weniger reaktiv als X ist.

19. Ein Verfahren nach Anspruch 16, worin die Verbindung der Formel II 2,4-Difluoronitrobenzol ist.

20. Eine Verbindung der Formel

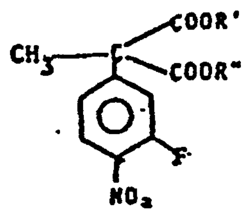

IIIa

worin R' und R'' wie in Anspruch 1 definiert oder eine Mono- bzw. Disäureform davon sind.

21. Diethyl 2-(3-Fluoro-4-Nitrophenyl)-2-Mehtylalonat

22. Eine Verbindung der Formel

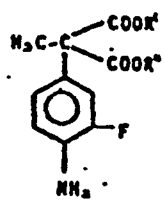

IVa

worin R' und R'' wie in Anspruch 1 definiert oder eine Mono-bzw. Disäureform davon sind.

23. Diethyl 2-(4-Amino-3-Flourophenyl)-2-Methylmalonat.